# EUROPÄISCHE PATENTANMELDUNG

(11) **EP 0 614 679 A2**
(43) Veröffentlichungstag der Anmeldung: **14.09.1994**
(21) Anmeldenummer: 94103428.2
(22) Anmeldetag: 07.03.1994
(51) Int. Cl.: A61N 5/06, F21V 9/12

(54) **Infrarotbestrahlungslampe mit einer im Strahlungsgang befindlichen Küvette**

(30) Priorität: 08.03.1993 DE 9303352 U
(71) Anmelder: MAXS AG, CH-6072 Sachseln (CH)
(72) Erfinder: Braun, Werner, CH-6062 Wilen-Sarnen (CH); Maner, Asim Dr., CH-6064 Kerns (CH); Rzeznik, Jerry, D-35452 Heuchelheim (DE)
(74) Vertreter: Grünecker, Kinkeldey, Stockmair & Schwanhäusser Anwaltssozietät

(57) **Zusammenfassung**

Bei einer Infrarotbestrahlungslampe (1) ist im Strahlengang eine Küvette (4) vorgesehen, die mit einer Flüssigkeit (11) gefüllt ist, welche Wasser und einen fungiziden Wirkstoff enthält. Die Küvette (4) besteht aber aus zwei sich gegenüberliegenden, transparenten Scheiben (10) und einem diese Scheiben (10) aufnehmenden Gehäuse (9). Die Flüssigkeit in der Küvette (4) ist eine Pufferlösung mit einem PH-Wert von 7, vorzugsweise auf Phosphatbasis.

## Beschreibung

Die Erfindung bezieht sich auf eine der Wärmetherapie dienende Infrarotbestrahlungslampe, mit einer im Strahlengang befindlichen, hermetisch abgeschlossenen Hohlraumküvette, die mit einer Flüssigkeit, die vorzugsweise Wasser und einen bakterizid und fungizid wirkenden Zusatz enthält, gefüllt ist.

Eine solche Bestrahlungslampe ist beispielsweise aus der EP 311 898 bekannt. Dort befindet sich am Gehäuseausgang, im Strahlengang zwischen dem Patienten und der Strahlungsquelle angeordnet, ein Filter, der zwei transparente planparallele Scheiben aufweist, die von einem Rahmen gehalten sind und einen geschlossenen Hohlraum begrenzen, der ein das Strahlungsspektrum selektiv beeinflußendes Medium enthält, das mit wenigstens einem Teil des Rahmens in Berührung steht. Da durch den Filtervorgang während des Betriebes der Filter stark erwärmt wird, ist zumeist eine Kühleinrichtung am Filter vorgesehen. Eine solche Kühleinrichtung können z.B. am Filter angebrachte, aus einem gut wärmeleitenden, wie z.B. Aluminium, gefertigte Kühlrippen sein.

Das von der Strahlungsquelle in seiner Gänze ausgesandte Strahlungsspektrum ist jedoch nicht nebenwirkungsfrei und damit für die Wärmetherapie nicht geeignet. Es enthält Spektrumanteile, die schon an der Hautoberfläche stark absorbiert werden, was zu einer für den Patienten unerträglichen Hitzeempfindung führt. Der Patient toleriert die Bestrahlung nicht und bricht die Behandlung vorzeitig ab, ehe eine therapeutisch wirksame Wärmedosis gebildet werden kann.

Mit der, im Strahlengang der Bestrahlungslampe angeordneten, gefüllten Küvette werden diese unerwünschten Spektrumsteile abgefangen und damit die geschilderten Nebenwirkungen beseitigt.

Als filterndes Medium kommt zumeist gereinigtes Wasser zum Einsatz, das die gewünschte spektrumformende Filterwirkung aufweist. Normales Leitungswasser findet dabei keine Verwendung, da die Menge der darin enthaltenen Fremdstoffe von Mal zu Mal variiert.
Ein zusätzliches Problem bereiten die häufig im Leitungswasser anzutreffenden Bakterien und Pilzsporen, die sich unter der Wärmeeinwirkung wuchernd vermehren und, durch die Stillstandzeiten unterstützt, rasch an Wachstum zunehmen. Dies führt sowohl im Hinblick auf das selektive Verhalten, als auch auf die Transparenz des Filters zur Verschlechterung des Filterwirkungsgrades, was neben der Überhitzung der Flüssigkeit in der Küvette, zwangsläufig zur Abnahme der therapeutischen Wirkung führt.

Um dem Effekt der Eintrübung des Filters durch Pilze und Bakterien vorzubeugen, werden dem Wasser üblicherweise Antiseptika und Fungizide beigemischt, die das Wasser klar und keimfrei halten sollen. Allerdings lassen sich Eintrübungen dennoch nicht ganz vermeiden.

Aufgabe der Erfindung ist es, eine gleichbleibend hohe, möglichst gute Filterwirkung der, mit Wasser gefüllten, Hohlraumküvette während des Betriebs der Bestrahlungslampe zu erreichen.

Die Aufgabe wird erfindungsgemäß dadurch gelöst, daß die Küvettenflüssigkeit aus Pufferlösung mit dem PH-Wert 7, vorzugsweise aus Phosphatbasis, besteht. Die Pufferlösung verhindert in Verbindung mit den fungiziden Wirkstoffen nunmehr die Eigeneintrübung der Flüssigkeit und führt somit zu einer gleichbleibend hohen Filterwirkung.

Als besonders günstig erweist sich das Zusetzen eines NaAgCl-Komplexes, der antiseptisch und fungizid wirkt. Dadurch wird der Bildung und Vermehrung von Schimmelpilzen und Bakterien und der daraus resultierenden Eintrübung des Küvetteninhalts entgegengewirkt. Es kann dabei ein NaAgCl-Komplex verwendet werden, der von der Fa. Katadyn-Produkte AG in CH-8394 Walliselen/Schweiz unter der Verwendung "Micropur MT 1" vertrieben wird.

Von besonderem Vorteil ist es dabei, eine Pufferlösung, basierend auf Kaliumhydrogenphosphaten zu verwenden, die mit dem NaAgCl-Komplex in vorteilhafter Weise zusammenwirkt. Es kann dabei eine "Pufferlösung gebrauchsfertig PH 7 +-0,02 (20°C)", die unter der Artikelnummer 9439.1 bzw. 9439.901 von der Fa. D. Merck in D-6100 Darmstadt im Handel vertrieben wird, Verwendung finden.

Überraschenderweise führt die Verwendung eines NaAgCl-Komplexes zusammen mit der Pufferlösung zu keinerlei Problemen bei Küvetten mit Aluminiumgehäusen, obwohl bei der Produktionsbeschreibung zu "Micropur MT 1" darauf hingewiesen wird, daß dieses Mittel für Aluminiumgefäße nur eingeschränkt geeignet ist.

Im folgenden wird ein Ausführungsbeispiel der Erfindung anhand einer Zeichnung naher erläutert. Es zeigen:
- Figur 1: eine schematische Darstellung der Anordnung mit Bestrahlungslampe und zu behandelnder Person
- Figur 2: eine schematische Schnittdarstellung des Details aus Figur 1.

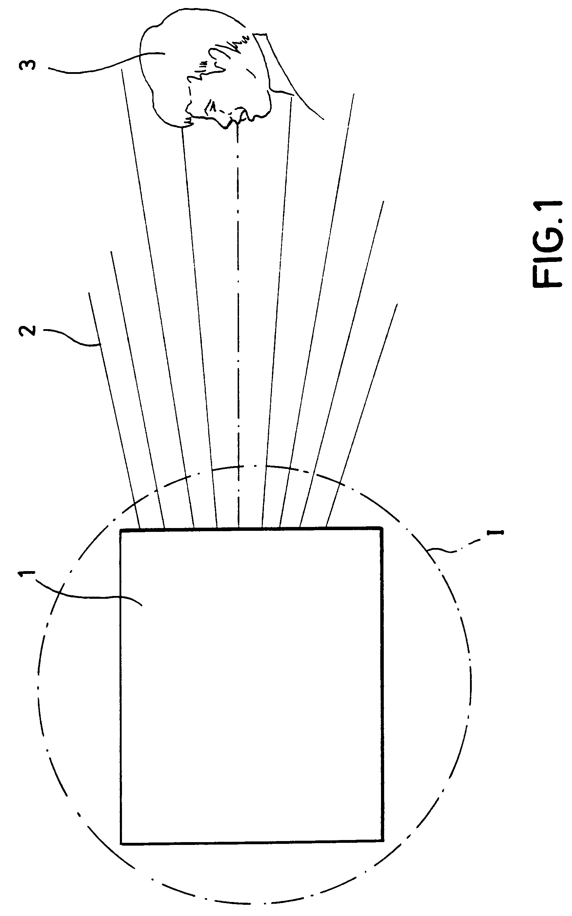

In Figur 1 ist die Gesamtanordnung mit einer Bestrahlungslampe 1 dargestellt, die Strahlen mit einem Strahlengang 2 emittiert. Im Strahlengang 2 befindet sich die zu behandelnde Person 3, wobei der zu behandelnde Teil der Person 3 im Strahlengang liegt und der Bestrahlungslampe 1 zugewandt ist. Zwischen der Person 3 und der Bestrahlungslampe 1 befindet sich im Strahlengang 2 eine Küvette 4.

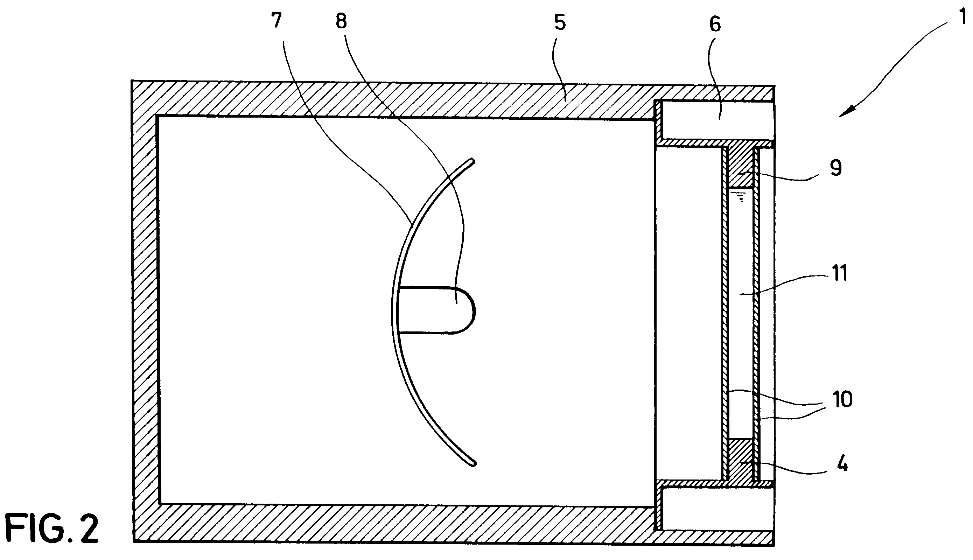

In Figur 2 ist die Bestrahlungslampe 1 mit einem Gehäuse 5 mit einer Öffnung 6, einem Reflektor 7, einer Strahlungsquelle 8 und der Küvette 4 dargestellt.

Die Küvette 4 besteht aus einem, aus Aluminium gefertigten Gehäuse 9, zwei Planparallelen, normal zum Strahlengang liegenden, wasserdicht angebrachten, transparenten Begrenzungen 10 und darin befindlichen, neutraliesierenden, Wasser 11 wobei dem Wasser eine neutralisierende Pufferlösung, die Kaliumhydrogenphosphat und Dinatriumhydrogenphosphat enthält und von der Fa. Merck in D-6100 Darmstadt, unter der Bezeichnung "Pufferlösung gebrauchsfertig PH 7,00 +-0,02 (20° C)" mit der Artikel-Nummer 9439.1 bzw. 9439.9010, vertrieben wird, beigemischt ist. Zusätzlich ist dem Wasser ein antiseptisch und fungizid wirkender NaAgCl-Komplex beigemischt, der von der Firma Katadyn Produkte AG in Zicha-8304 Wallisellen/Schweiz unter der Bezeichnung "Micropur MT1" vertrieben wird.

Im folgenden wird die Wirkungs- und Funktionsweise der Erfindung näher erläutert.

Die von der Strahlenquelle 8 emittierte Strahlung wird durch den Reflektor 7 durch die Öffnung 6 aus dem Gehäuse 5 geleitet und trifft auf die Küvette 4. In der Küvette 4 filtert das, in der Küvette 4 befindliche, Wasser 11 die für die Behandlung unerwünschten Wellenlängen aus der, von der Strahlungsquelle 8 emittierten Strahlung. Anschließend tritt die verbleibende Strahlung aus der Küvette aus und trifft auf die Person, wobei die Strahlung ihre therapeutische Wirkung entfaltet.

Der im Wasser 11 befindliche antiseptisch und fungizid wirkender NaAgCl-Komplex verhindert die Bildung und das Ausbreiten von Bakterien und Schimmelpilzen. Dabei wird die Zunahme der Verbreiterung der Absorptionsbanden verhindert, was im Nebeneffekt zur erwünschten Verringerung der Wärmeentwicklung im Küvetteninhalt führt.

## Patentansprüche

1. Infrarotbestrahlungslampe, mit einer im Strahlungsgang befindlichlichen Küvette (4), die mit einer Wasser und einem fungiziden Wirkstoff enthaltenden Flüssigkeit (11) gefüllt ist, zur Wärmebehandlung des menschlichen Körpers, wobei die Küvette (4) aus zwei sich gegenüberliegenden, transparenten Scheiben (10) und einem, diese Scheiben (10) aufnehmendem Gehäuse (9), besteht, **dadurch gekennzeichnet**, daß die Flüssigkeit (11) eine Pufferlösung mit dem PH-Wert 7, vorzugsweise auf Phosphatbasis, ist.

2. Infrarotbestrahlungslampe nach Anspruch 1, **dadurch gekennzeichnet**, daß der fungizide Wirkstoff ein NaAgCl-Komplex ist.

3. Infrarotbestrahlungslampe nach Anspruch 1 oder 2, **dadurch gekennzeichnet**, daß die Pufferlösung (11) auf Kaliumhydrogenphosphaten basierend ist.
